(19) Europäisches Patentamt
European Patent Office
Office européen des brevets

(11) **EP 1 834 583 B1**

(12) **FASCICULE DE BREVET EUROPEEN**

(45) Date de publication et mention
de la délivrance du brevet:
**19.11.2008   Bulletin 2008/47**

(51) Int Cl.:
***A61B 5/22*** *(2006.01)*

(21) Numéro de dépôt: **06111352.8**

(22) Date de dépôt: **17.03.2006**

(54) **Procédé et dispositif d'évaluation des capacités musculaires d'athlètes à l'aide de tests brefs**

Vorrichtung und Verfahren zur Auswertung der muskularischen Kapazität unter Verwendung von kurzen Tests

Methods and device for the evaluation of muscular capacity of athletes with short tests

(84) Etats contractants désignés:
**AT BE BG CH CY CZ DE DK EE ES FI FR GB GR HU IE IS IT LI LT LU LV MC NL PL PT RO SE SI SK TR**

(43) Date de publication de la demande:
**19.09.2007   Bulletin 2007/38**

(73) Titulaire: **Myotest SA**
**1950 Sion (CH)**

(72) Inventeurs:
• **Flaction, Patrick**
**1965 Savièse (CH)**
• **Praz, Manu**
**1994 Baar (CH)**

(74) Mandataire: **P&TS**
**Patents & Technology Surveys SA**
**Rue des Terreaux 7**
**P.O.Box 2848**
**2001 Neuchâtel (CH)**

(56) Documents cités:
**US-A- 5 056 783          US-A- 5 474 083**
**US-A1- 2004 134 274     US-B1- 6 397 151**

## Description

### Domaine technique

La présente invention concerne le domaine des procédés et des dispositifs d'évaluation de la capacité musculaire, notamment pour le sport et la réhabilitation. La présente invention concerne en particulier les dispositifs d'évaluation des valeurs physiologiques musculaires, par exemple de la force et la puissance musculaire, basés sur des mesures d'accélération.

### Etat de la technique

L'entraînement sportif des athlètes et la réhabilitation après accident font de plus en plus souvent appel à des appareils de mesure de performance. Par exemple, en entraînement cardiovasculaire (jogging, vélo, etc), l'usage de dispositifs de mesure de la fréquence cardiaque (« pulsomètres ») et de pédomètres s'est largement répandu depuis quelques années. Ces appareils portables effectuent des mesures au cours de l'effort qui permettent à l'athlète d'adapter l'entraînement à l'aide de données objectives. Les grandeurs fournies indiquent typiquement le rythme cardiaque, la distance parcourue, la durée d'entraînement, la vitesse moyenne ou maximale, etc. Ils ne fournissent cependant aucune mesure directe de la puissance musculaire de l'athlète ou d'une chaîne musculaire de l'athlète.

US5788655 (Omron) décrit un appareil destiné à être fixé sur le corps et muni d'un accéléromètre et d'un afficheur LCD. L'appareil mesure en permanence les déplacements du porteur pour déterminer son niveau d'activité physique et des d'autres grandeurs dépendant du métabolisme, par exemple la consommation de calorie journalière de l'utilisateur. Ce type d'appareil est utile pour mesurer de manière plus objective le niveau de sédentarité de patients. Il est cependant inadapté à l'entraînement musculaire et à la mesure d'efforts brefs, et ne permet pas de déterminer par exemple la puissance maximale d'un groupe musculaire de l'athlète.

WO2005074795 (Nokia) décrit un terminal de mesure muni d'un accéléromètre et attaché sur le corps d'un athlète. Les données de mesure sont évaluées pour fournir une grandeur représentative de l'intensité de l'effort fourni. A nouveau, le but est de déterminer le niveau d'activité sur une longue période, par exemple une journée ou une semaine.

WO03/032826 (Philips) décrit un système comparable et muni d'un accéléromètre à trois axes pour déterminer le niveau d'activité physique d'un patient. Le dispositif proposé affiche des grandeurs telles que le taux métabolique quotidien, la dépense énergétique quotidienne ou la dépense d'énergie induite par l'exercice. Cet appareil permet donc utile de mesurer des accélérations sur une période de plusieurs heures, voire de plusieurs jours.

Les dispositifs du type décrit ci-dessus conviennent donc essentiellement à la mesure d'activité au cours d'exercices longs, par exemple au cours d'une séance de jogging, d'un match de badminton ou d'une journée ordinaire. Il existe aussi des dispositifs assez similaires pour détecter les chutes de personnes âgées, le temps qu'elles passent assis, debout ou couchées, etc.

Il existe cependant aussi un besoin pour la mesure de paramètres au cours d'efforts musculaires brefs ou même très brefs. Par exemple, l'entraînement musculaire et la mesure de la capacité musculaire des athlètes font souvent appel à des mouvements très courts, par exemple un seul lever de charge sur une machine de musculation, ou un saut en hauteur. La fréquence d'échantillonnage employée par les dispositifs ci-dessus ne permet généralement pas de les employer à la mesure de paramètres cinématiques au cours d'efforts aussi brefs. Par ailleurs, les données calculées à partir des accélérations mesurées sont de peu d'intérêt pour des efforts brefs ; il est par exemple peu utile de connaître la quantité de calories brûlées au cours d'un seul mouvement. Il existe en revanche un besoin pour des appareils adaptés notamment à la musculation, qui permettent de mesurer par exemple le gain de force ou de puissance sur un muscle ou un groupe musculaire entraîné.

On connaît déjà dans l'art antérieur certains dispositifs de mesure spécifiquement destinés à l'entraînement et la mesure de mouvements brefs. Par exemple, US5474083 décrit un système destiné à surveiller les mouvements de levé de charge par un patient. Le système emploie des électrodes pour mesurer l'activité des muscles du patient au cours du mouvement, ainsi qu'un détecteur de mouvement de la charge. Une alarme est déclenchée en cas de mouvement inapproprié. Ce système est utile pour empêcher les accidents provoqués par le lever de charges incorrect, ou pour exercer des personnes à soulever des charges sans se blesser. Il est cependant inapproprié à la mesure de la performance musculaire du sportif. Par ailleurs, l'usage d'électrodes rend son emploi peu pratique.

US6148280 (Virtual Technologies) décrit un dispositif muni d'accéléromètres et de gyroscopes disposés sur tout le corps d'un athlète. Les données fournies par plusieurs capteurs sont transmises à un ordinateur personnel qui permet d'analyser la trajectoire et d'autres caractéristiques du mouvement. Ce système est complexe, car il met en oeuvre plusieurs capteurs, y compris des gonomiètres coûteux, relativement fragiles. La connexion des capteurs entre eux et vers l'ordinateur externe renchérit le dispositif, et rend son installation malaisée. Il est adapté à l'entraînement de mouvements précis, par exemple un swing de golf, mais ne permet pas de déterminer directement la capacité musculaire développée par le sportif au cours de ce mouvement.

DE446302 décrit un accéléromètre utilisé dans les sports de combat pour la mesure de l'accélération de la surface de frappe. L'appareil n'est pas portable et convient uniquement aux sports de combat tels que box, karaté, etc. Un ordinateur externe doit être employé pour évaluer les résultats de mesure et les afficher.

US 6 397 151 (Casio Computer Co.) décrit un dispositif portable sous la forme d'une montre comprenant un accéléromètre utilisé pour la mesure de l'accélération de frappe.

D'autres dispositifs basés sur des accéléromètres et des gyroscopes existent qui permettent par exemple de vérifier la trajectoire d'un swing au golf, afin d'améliorer le mouvement. Ce type de dispositifs fournit un grand nombre de données, par exemple la position et la vitesse du capteur en chaque instant, qui nécessitent souvent un écran de grande taille ou un dispositif externe pour les visualiser. Ils ne permettent cependant pas de calculer et d'afficher immédiatement sur le dispositif une ou plusieurs grandeurs représentatives de la capacité musculaire de l'athlète.

Bref résumé de l'invention

La présente invention a pour but un procédé et un dispositif de mesure permettant d'évaluer les capacités musculaires des athlètes. La présente invention vise à proposer un test rapide avec un instrument simple à utiliser, autonome, économique, et apte à fournir immédiatement des données représentatives des qualités musculaires des athlètes, par exemple de leur explosivité, puissance, détente musculaire, etc.

Un but de la présente invention est de proposer un appareil de mesure de performance sportive spécifiquement destiné à l'entraînement et à la réhabilitation musculaire, en faisant par exemple appel à des tests musculaires connus comme des sauts ou de levers de charge. En particulier, le dispositif doit permettre d'évaluer la détente, l'explosivité, le taux d'accroissement de force (TAF) des athlètes, au moyen de tests brefs et non invasif.

Selon l'invention, ces buts sont notamment atteints au moyen d'un procédé d'évaluation des capacités musculaires d'athlètes à l'aide de tests brefs tels que levers et/ou sauts, comprenant les étapes suivantes :

fixation à une charge en mouvement au cours du test d'un dispositif de mesure amovible et autonome électriquement, ledit dispositif de mesure étant basé sur un accéléromètre tri-axial,
détermination d'une séquence de valeurs d'accélération successive de ladite charge au cours dudit test,
au terme dudit test, indication sur ledit affichage d'au moins une grandeur représentative de ladite capacité musculaire et déterminée à partir de ladite séquence de valeurs d'accélération.

La capacité musculaire peut être calculée et affichée au terme du test par exemple sous la forme de puissance maximale, de taux d'accroissement de la force, etc.

Le dispositif est adapté à la mesure de l'accélération en fonction du temps, et à l'évaluation des résultats, par exemple lors de l'exécution des tests suivants :

lever de charge ;
squat jump et/ou countermovement jump ;
drop jump.

Le calcul des grandeurs affichées tient compte de la connaissance a priori de la forme de la fonction d'accélération au cours de ces tests musculaires standardisés. Le dispositif utilise cette connaissance préalable pour déterminer des grandeurs qu'un dispositif générique ne pourrait pas mesurer. Par exemple, le dispositif de l'invention peut dans un mode de réalisation décomposer un mouvement en phases clés et calculer la puissance maximale au cours d'une phase particulière. Par exemple, un saut peut comporter une phase d'extension musculaire suivie d'une phase de flexion ; le dispositif peut segmenter les données mesurées pour déterminer le début et la fin de ces deux phases, puis calculer par exemple la puissance lors de l'extension et la vitesse maximale lors de la flexion.

Typiquement, le dispositif doit être capable d'acquérir des données d'accélération selon trois axes, au moins tous les 1/100e de seconde, au cours d'un effort bref, c'est-à-dire un effort dont la durée est typiquement inférieure à 10 secondes, mais peut éventuellement aller jusqu'à plusieurs minutes.

L'utilisation d'un accéléromètre à trois axes permet de calculer l'accélération selon n'importe quelle direction, notamment selon la direction de déplacement de la charge, par exemple la direction verticale lors d'un saut. Dans une variante préférentielle, seule une séquence d'accélérations selon cette direction préférentielle est utilisée pour le calcul des grandeurs affichées.

Brève description des dessins

Des exemples de mise en oeuvre de l'invention sont indiqués dans la description illustrée par les figures annexées dans lesquelles :

La figure 1 illustre de manières schématiques différentes phases des mouvements d'un athlète au cours d'un lever de charge de type « développer-couché ».

La figure 2 illustre la hauteur de la charge soulevée en fonction du temps (h(t)) au cours de l'exercice de « développer-couché » illustré sur la figure 1.

La figure 3 illustre la vitesse de la charge soulevée en fonction du temps (v(t)) au cours de l'exercice de « développer-couché » illustré sur la figure 1.

La figure 4 illustre l'accélération de la charge soulevée en fonction du temps (a(t)) au cours de l'exercice de « développer-couché » illustré sur la figure 1.

La figure 5 illustre la force maximale, la vitesse maximale et la puissance maximale que peut déployer un athlète en fonction de la charge déplacée.

La figure 6 illustre l'accélération a(t) en fonction du temps au cours d'un exercice.

La figure 7 illustre un test de squat jump.

La figure 8 illustre un test de countermovement jump.

La figure 9 illustre un test de drop jump.

La figure 10 illustre un dispositif d'évaluation de capacité musculaire selon l'invention.

La figure 11 est un schéma-bloc des principaux composants électroniques du dispositif de la figure 10.

La figure 12 est un diagramme de flux illustrant le paramétrage du dispositif.

La figure 13 est un diagramme de flux illustrant le test de capacité musculaire au cours du procédé

Exemple(s) de mode de réalisation de l'invention

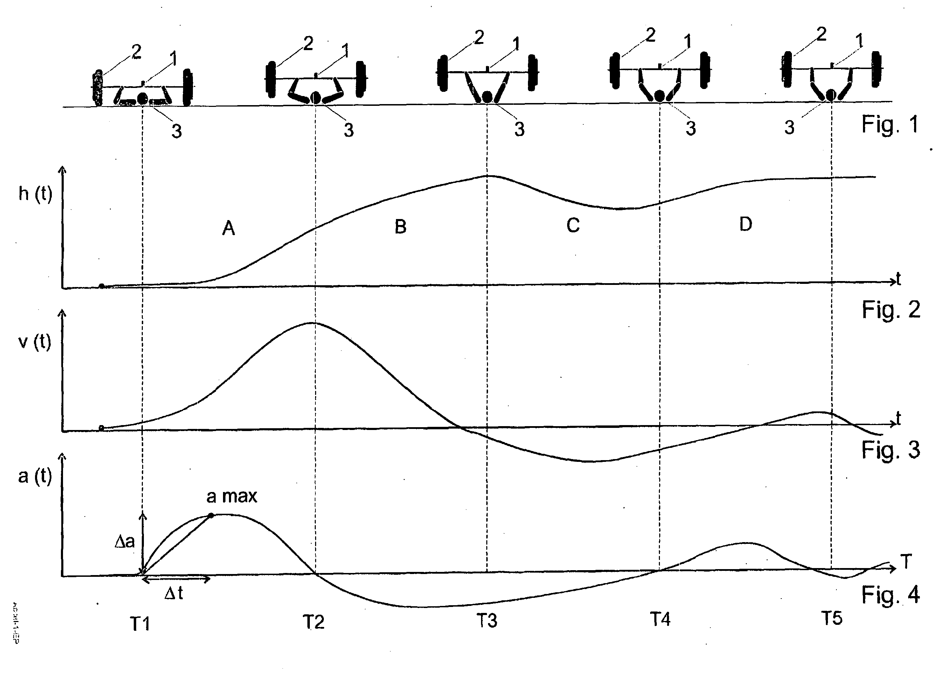

Les figures 1 à 4 illustrent l'évolution de différents paramètres cinématiques au cours d'un mouvement de lever de charge de type « développer-couché ». Ce mouvement, très utilisé en musculation, consiste à soulever une charge 2 avec les deux bras, à partir d'une position couchée sur le dos. La charge est soulevée le plus haut possible en combinant une adduction de l'épaule et une extension du coude. La figure 1 illustre cinq instants clés du test. Le test démarre en T1, dans la position initiale illustré sur la première image à gauche de la figure 1. La charge est à son point le plus bas, les coudes de l'athlète 3 sont pliés.

Au cours de la phase A, entre les instants T1 et T2, l'athlète 3 soulève la charge dont la vitesse augmente constamment, comme indiqué sur les figures 2 à 4. La force de poussée déployée au cours de cette phase est maximale et les bras s'étendent.

Au cours de la phase B, entre les instants T2 et T3, la poussée se poursuit, mais la vitesse de lever diminue ; l'accélération devient donc négative, comme on le voit sur la figure 4. La hauteur de la charge est maximale au point T2.

L'athlète relâche ensuite son effort au cours de la phase C entre les instants-clés T3 et T4. La charge redescend légèrement, en sorte que sa vitesse devient négative. Cette étape est suivie par une phase D de stabilisation, entre les instants T4 et T5, au cours de laquelle l'athlète maintient ses bras en extension, mais tend à abaisser les épaules. L'accélération subie par la charge 2 au cours de cette phase tend progressivement vers 1 G (gravitation terrestre).

Les instants-clés T1 à T5 peuvent être déterminés sans ambiguïtés à partir des données a(t), v(t) et h(t). Comme on le verra, ces grandeurs peuvent être mesurées au cours de l'effort au moyen d'un dispositif 1 lié à la charge, placé idéalement au centre de masse ; elles seront utilisées pour déterminer et afficher la capacité musculaire de l'athlète.

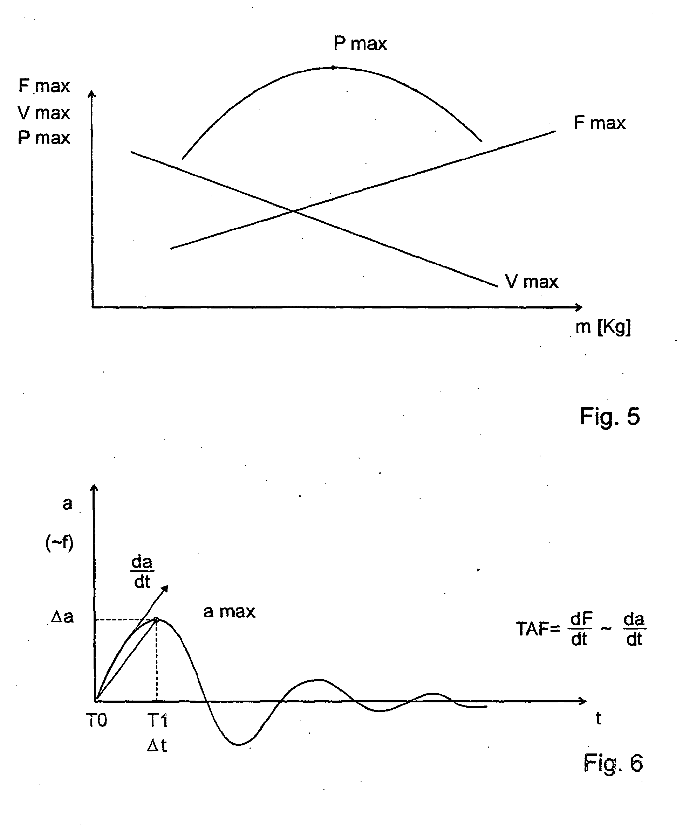

La figure 5 illustre de manière schématique une évolution possible de la force maximale $F_{max}$, de la vitesse maximale $V_{max}$ et de la puissance maximale $P_{max}$ que peut déployer un athlète soulevant une charge de masse variable. La force gravitationnelle (m · g) exercée sur la charge déployée augmente linéairement avec la masse de la charge.

Par contre la force (m · a) exercée par l'athlète pour arracher cette masse à la gravité dépend directement de la force de l'athlète dans le déplacement de la charge. L'athlète a en effet la possibilité de modifier l'accélération imposée à la charge.

La vitesse de lever maximale $V_{max}$ tend à diminuer avec l'augmentation de la masse soulevée ; l'athlète soulève plus rapidement des masses légères. La puissance maximale P déployée par l'athlète au cours de l'effort est déterminée par la formule :

$$P = \frac{dE}{dt} = F \cdot v = m \cdot a \cdot v$$

Cette formule est valable dans le cas où l'accélération et la vitesse sont parallèles, par exemple lors de déplacements purement verticaux. En cas de vecteurs non parallèles, le calcul doit être effectué vectoriellement.

La puissance maximale $P_{max}$ au cours de l'effort passe généralement par un optimum pour une valeur donnée de m. Une barre très lourde pour un athlète va l'engager essentiellement dans des contraintes d'opposition, pour s'opposer à la force gravitationnelle mg, et peu dans des contraintes d'accélération ma (la barre s'élève plus lentement). On voit ainsi que la connaissance de la puissance maximale, telle qu'elle peut être obtenue en mesurant l'accélération et la vitesse de la charge, permet de déterminer la charge optimale avec laquelle doit s'exercer un athlète pour maximiser la puissance déployée lors de l'exercice.

La figure 6 illustre la variation d'accélération a (t) au cours du temps lors d'un effort bref typique, par exemple un lever ou un saut. Au cours de la première phase de durée ΔT entre les instants T0 et T1, l'accélération augmente de Δa. Si la masse soulevée est constante, l'accélération est proportionnelle à la force. On a donc que le taux d'augmentation de la force (TAF, ou explosivité), employé en mesure sportive, est simplement proportionnel à Δa/Δt. Le taux d'augmentation de la force est également proportionnel à la puissance déployée par l'athlète, du moins lorsque la distance h parcourue par la charge est constante.

Il est également possible de déterminer la puissance instantanée P(t) à partir de la valeur da/dt en chaque point de la courbe a(t), et de déterminer la puissance maximale $P_{max}(t) = \max (P(t))$. Cette valeur est cependant plus sensible aux erreurs et au bruit de mesure ; un lissage de la courbe a(t) à l'aide d'un filtre anti-bruit est utile pour réduire les influences parasites.

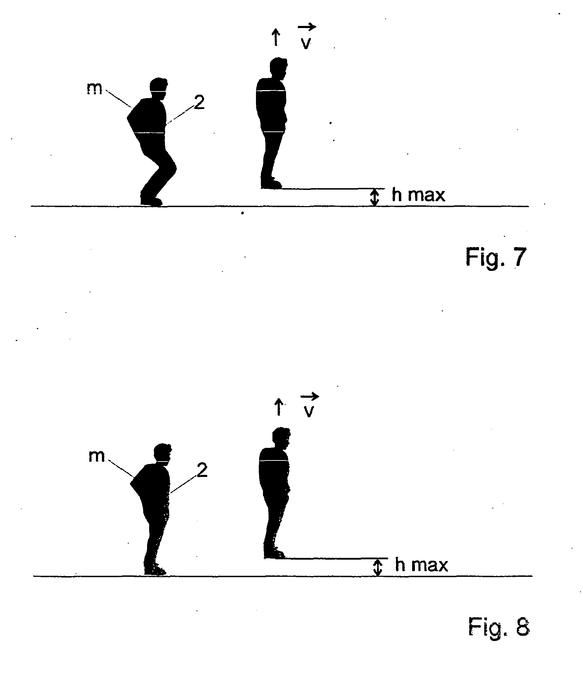

La figure 7 illustre un autre test employé pour déterminer la capacité musculaire d'un athlète 3. Ce test, appelé « squat jump », vise notamment à évaluer la détente des athlètes, en particulier la détente sèche, sans étirement, et leur aptitude à développer beaucoup de force en un temps très court (explosivité). L'athlète part avec les genoux fléchis à 90° et les mains sur les hanches, puis tente de sauter le plus haut possible. Une bonne accélération initiale est indispensable pour atteindre une hauteur élevée.

Un test similaire, le counter-movement jump, est illustré sur la figure 8. Dans ce test, l'athlète part avec les jambes tendues et est autorisé à effectuer une flexion avant l'extension et le saut. La différence entre le résultat obtenu en squat jump et la hauteur normalement plus élevée obtenue en counter-movement jump dépend de la qualité d'élasticité musculaire de l'athlète et/ou de sa capacité à développer une force plus importante lors de l'amortissement.

Des tests similaires peuvent être effectués en autorisant l'athlète à s'aider des bras lors du saut, afin notamment de vérifier la coordination des bras et des jambes. La qualité de rebond peut être mesurée par des multisauts, généralement effectués sur place, avec l'aide des bras. On effectue parfois aussi des tests de squat jump et de counter-movement jump avec une charge sur les épaules de l'athlète.

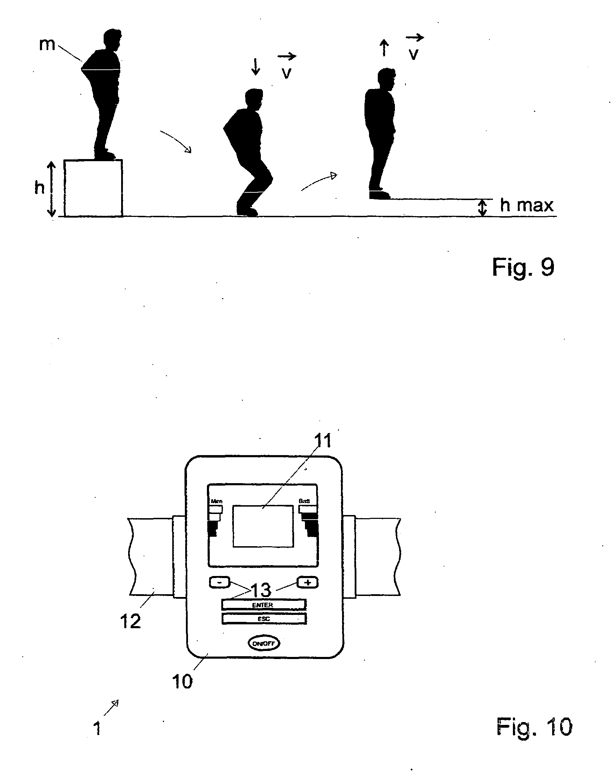

Le drop jump illustré sur la figure 9 consiste en un saut en hauteur après une chute depuis une hauteur connue. L'impulsion est donc précédée d'une mise en tension importante qui provoque l'allongement des tendons et une sollicitation musculaire différente. Les meilleurs athlètes augmentent ainsi leur performance de saut. En général on effectue le test à 20 cm, 40 cm, 60 et 80 cm de chute pour déterminer la bonne hauteur de travail pour chaque sujet.

Les sauts évoqués ci-dessus peuvent être décomposés en phases distinctes, séparées par des instants clés. Typiquement, un squat jump comporte les phases suivantes : départ, appel, impulsion, vol, contact, amorti au retour, fin.

Les tests ci-dessus peuvent être répétés, par exemple en effectuant une série de 10 sauts ou de 20 levers, afin de mesurer par exemple l'évolution des performances musculaires et la résistance à la fatigue de l'athlète. Il est aussi possible d'effectuer des mesures d'asymétrie musculaire, sur les bras ou les jambes, par exemple en effectuant d'abord un lever avec le bras ou la jambe gauche, puis avec le membre droit correspondant. Enfin, certains tests permettent de mesurer des disbalances entre muscles ou groupes de muscles, par exemple une différence de force anormale entre biceps et triceps. Ces différents tests impliquent plusieurs mesures consécutives qui peuvent être stockées en mémoire puis comparées entre elles. Le résultat de la comparaison peut être affiché par exemple sous forme de pourcentage, par exemple 20% de différence de puissance entre jambe gauche et jambe droite. Dans l'art antérieur, les performances des athlètes dans les tests de saut décrits ci-dessus sont souvent déterminés à l'aide d'un tapis de contact qui déclenche un chronomètre lorsque l'athlète quitte le tapis, et l'interrompt lorsqu'il retourne sur le sol. Le temps de suspension permet d'évaluer la hauteur atteinte par l'athlète. Ces dispositifs sont cependant encombrants, coûteux et permettent d'obtenir les données recherchées uniquement de manière indirectes, à partir de mesures de durées.

Selon le procédé de l'invention, une mesure plus significative est obtenue en associant à l'athlète 3 un dispositif 1 muni d'un accéléromètre tri-axe, qui permet de mesurer l'accélération verticale de l'athlète au cours du lever de charge ou du saut.

Un exemple de dispositif 1 approprié est illustré sur la figure 10. Le dispositif 1 comporte un boîtier, par exemple un boîtier en plastique de moins de 100 grammes (y compris le contenu) que l'athlète peut fixer, selon l'exercice effectué, sur son corps ou sur la charge soulevée à l'aide de moyens de fixation 12, par exemple d'une bande velcro, d'une sangle, etc. Le boîtier est de préférence étanche et permet une utilisation en plein air et en résistant à la sueur de l'athlète. Les moyens de fixation 12 comportent de préférence une ceinture pour fixer le dispositif près du centre de masse de l'athlète, par exemple à la hanche, à la taille ou de préférence au niveau du sacrum, dans une position peu inclinée lors de l'effort. Des essais ont en effet montré qu'une ceinture 12 arrangée pour un porter du dispositif sur le sacrum permet de minimiser l'influence des mouvements du tronc dans la direction antéropostérieure. La largeur maximale de la ceinture est de préférence importante, par exemple supérieure à 10 centimètres, afin d'améliorer son maintien et d'éviter qu'elle ne soit entraînée par les mouvements de la peau et des chairs sur le squelette.

Dans une variante, les moyens de fixation permettent de fixer le dispositif sur une ceinture existante. Il est cependant indispensable de veiller à employer une ceinture qui ne bouge pas lors des exercices. Une fixation au poignet est en revanche peu adaptée pour la plupart des tests de musculation, notamment les tests décrits plus haut, parce que les accélérations des bras ou du poignet s'ajoutent aux accélérations du corps, et parce que le poignet ne reste pas vertical au cours de la plupart des efforts.

Le dispositif comporte en outre un affichage 11, par exemple un écran à cristaux liquides alphanumérique ou matriciel, qui permet d'afficher des menus de commande, l'état de la mémoire, de la batterie, ainsi que les grandeurs numériques déterminées au cours ou à l'issue du test. Des organes de commande 13, par exemple des boutons et/ou des organes de déplacement de curseur permettent de naviguer dans les menus affichés, de sélectionner des options, d'introduire des données, et de sélectionner les résultats affichés.

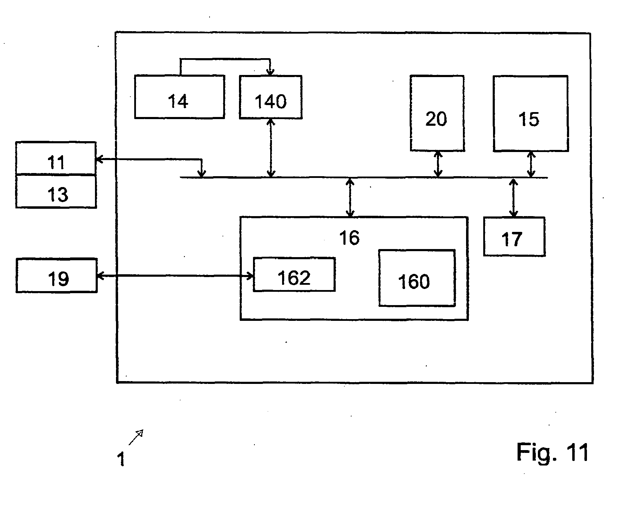

La figure 11 est un schéma-bloc illustrant les principaux composants électroniques du dispositif 1. Outre les organes externes déjà décrits en relation avec la figure 10, il comporte un accéléromètre tri-axial 14, par exemple un accéléromètre réalisé sous forme de composant MEMS et lié à un convertisseur analogique-numérique 140, ou intégrant directement un tel convertisseur, pour fournir des séquences de mesures d'accélération selon trois axes. L'accéléromètre 14 peut comporter un ou plusieurs axes privilégiés, offrant une précision ou une résolution de mesure supérieure aux autres axes. Cet axe privilégié sera de préférence aligné verticalement lorsque le dispositif se trouve dans sa position d'utilisation habituelle, afin d'améliorer le mesure selon la direction verticale. De préférence, le dispositif est dépourvu de gyroscope afin de réduire son coût, sa consommation et le volume de données produites ; l'usage d'un gyroscope à un axe, ou même à trois axes, pourrait cependant être envisagé pour certains types de test de capacité musculaire, ou pour calibrer de manière plus certaine la position verticale.

Le dispositif 1 est de préférence autonome électriquement et alimenté par exemple à l'aide d'une batterie 15 ou d'un accumulateur rechargeable, par exemple au travers de la prise USB 19 ou en le sortant du boîtier. La batterie 15 alimente notamment un microprocesseur 16 ou un microcontrôleur muni d'une mémoire 160 RAM et/ou EEPROM. Le microprocesseur exécute un programme de préférence chargé en EEPROM, et remplaçable au travers d'une des interfaces, pour analyser les données de mesure fournies par l'accéléromètre 14 et commander l'affichage 11 afin d'afficher les grandeurs désirées.

Le dispositif 1 comporte encore une horloge en temps réel (RTC) 20 qui permet notamment de mesurer des intervalles de temps $\Delta t$, ainsi qu'un buzzer 17 ou un haut-parleur pour générer des signaux d'alarme ou d'autres sons. Un module d'entrée-sortie (UART) 162 permet au microprocesseur 16 d'échanger des données avec des dispositifs externes, par exemple pour le reprogrammer ou pour transmettre des résultats de mesure à un ordinateur personnel, un téléphone mobile ou un autre dispositif externe de traitement de données. Le module 162 permet également d'introduire en tout temps les paramètres de nouveaux types de test, et de déterminer la manière dont les données de mesure pour ces nouveaux tests seront exploitées pour en extraire les grandeurs représentatives souhaitées.

Le module 162 est lié à une interface 19, par exemple une prise USB, une interface sans-fil de type Bluetooth ou autres, etc.

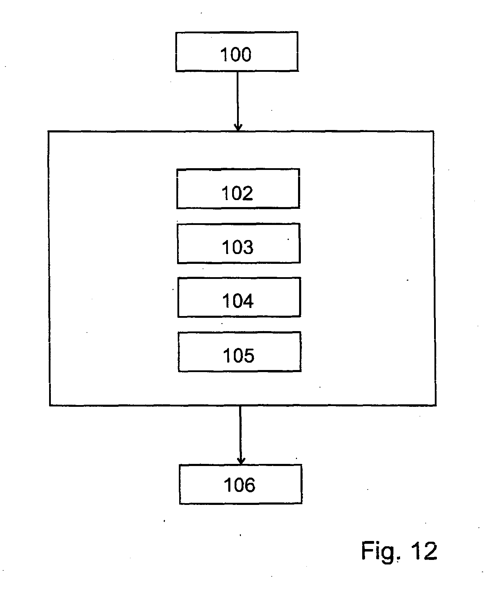

La figure 12 est un diagramme de flux du programme « setup » qui est automatiquement exécuté lors de la première utilisation du dispositif, ou sur appel de l'utilisateur. Le programme setup est de préférence mémorisé dans la mémoire EEPROM 160 et exécuté par le microprocesseur 16. Il démarre à l'étape 100 avant d'exécuter la routine 101 au cours de laquelle l'utilisateur a la possibilité d'introduire des informations stockées de manière permanente dans le dispositif. Au cours de l'étape 102, l'utilisateur peut introduire son identification (UserId ou nom) et éventuellement un mot de passe optionnel pour rendre le dispositif inutilisable pour un éventuel voleur. Au cours de l'étape 103, il peut introduire les unités de mesure préférées (kg/cm ou lb/inch), qui lui permettent de définir lors de l'étape 103 sa masse employée pour le calcul de la force, du travail et de la puissance dans les exercices de saut notamment.

L'utilisateur peut ensuite introduire la date et l'heure actuelle, qui seront ensuite modifiées en permanence par l'horloge 20.

D'autres données permanentes pouvant être introduites à ce stade comprennent par exemple la langue préférées des menus, le type de résultat affiché, le type d'exercice par défaut, etc.

Les données numériques peuvent être introduites par exemple par incrémentation de valeurs au moyen des touches + et -, ou à l'aide d'un clavier numérique non représenté, ou par n'importe quel autre moyen d'entrée de données approprié.

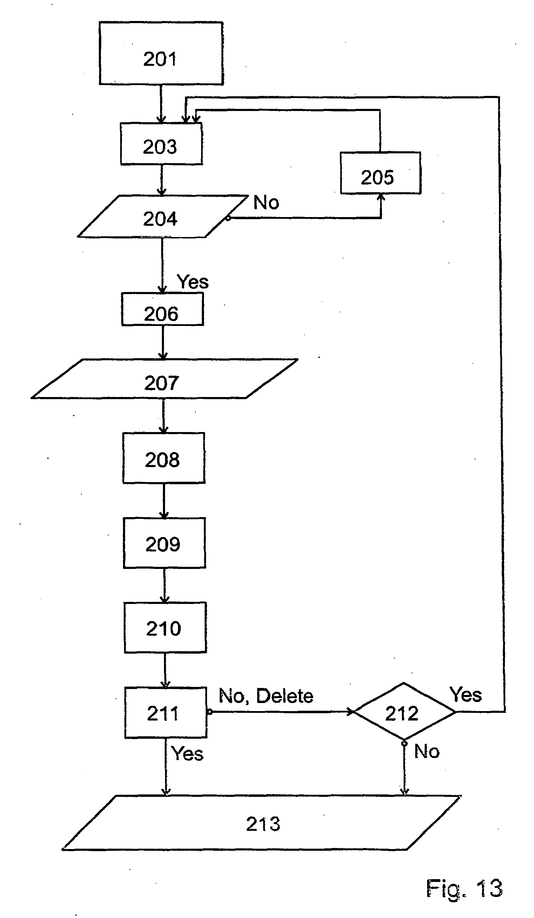

La procédure de setup se termine à l'étape 106, et passe de préférence immédiatement en mode de test pour tester les capacités musculaires de l'athlète. La procédure de test est illustrée avec le diagramme de flux de la figure 13.

Au cours de l'étape 201, l'utilisateur est invité à choisir dans un menu le type de test qu'il souhaite effectuer. Cette étape peut être implicite si un choix par défaut est mémorisé dans les paramètres permanents du dispositif ; il est aussi possible de proposer par défaut le dernier test effectué. Dans une variante préférentielle, l'utilisateur est invité à choisir parmi entre les options suivantes, ou à confirmer un choix proposé parmi ces options :

    1. Lever de charge

    2. Saut (squat jump ou countermovement jump)

    3. Drop jump.

L'utilisateur est aussi invité à sélectionner ou à confirmer des paramètres dépendant du type de test. En cas de lever, il devra ainsi indiquer le poids de la charge, dans l'unité par défaut du dispositif. En cas de saut, il pourra être amené à confirmer son poids. En cas de drop jump, le dispositif l'invitera à introduire ou à confirmer la hauteur de saut, ainsi que son propre poids.

Le test proprement dit commence à l'étape 203, au cours de laquelle l'utilisateur (l'athlète) presse une touche, par exemple une touche « ENTER » ou « START » (non représentée). Le dispositif détermine ensuite au cours de l'étape de calibration 204 son orientation par rapport à la verticale. Pour cela, l'athlète est invité par exemple par un message sur l'affichage 11 à se tenir immobile pendant un intervalle, par exemple au moins deux secondes. Dans cette position, seule la gravitation impose une accélération sur l'accéléromètre triaxial 14. Une matrice de conversion de référentiel est calculée au cours de cette étape pour convertir les mesures des trois axes dans un référentiel correctement orienté par rapport à la verticale. Si l'accéléromètre détecte que l'utilisateur bouge durant l'intervalle de calibration 204, la calibration est refusée, et un message d'erreur visuel et/ou sonore est émis sur l'affichage 11 et/ou par le buzzer 17. L'utilisateur est alors invité à recommencer la calibration. Il peut en aller de même si l'orientation de l'appareil est trop inclinée par rapport à la position verticale idéale, par exemple si la mesure de l'accélération verticale dépend trop largement des résultats des axes non préférentiels de l'accéléromètre.

D'autres méthodes de calibration et de détermination de la position verticale peuvent être envisagées, y compris des méthodes basées sur une action de l'utilisateur qui peut être invité à poser le dispositif sur une surface rigoureusement horizontale, ou contre un mur vertical, ou des procédés mettant en oeuvre des capteurs supplémentaires, par exemple un goniomètre pour déterminer l'inclinaison du corps de l'athlète, un gyroscope multi-axes, un géorécepteur satellitaire de type GPS, etc.

En cas de calibration réussie, un bip sonore est émis au cours de l'étape 206 pour inviter l'utilisateur à effectuer le test. Le signal sonore produit peut dépendre du test choisi et peut en outre être accompagné d'un message sur l'affichage 11. L'acquisition d'une séquence de données d'accélération commence ensuite immédiatement à l'étape 207 et se termine à l'étape 208, après une durée prédéterminée (par exemple 10 secondes), lorsque la mémoire du dispositif est pleine ou lorsque l'utilisateur appuie sur une touche du dispositif, par exemple une touche marquée « END » ou « START/STOP ». Il est aussi possible d'interrompre l'acquisition des données automatiquement en fonction des données mesurées, par exemple lorsque le dispositif détermine que l'athlète a rejoint le sol après un saut et que son accélération verticale est à nouveau nulle ou égale à 1G.

L'accélération est de préférence mesurée selon trois axes, au mois tous les 100$^e$ de secondes ou de préférence toutes les 2 millisecondes. Les trois séquences de valeur d'accélération sont stockées dans la mémoire du microprocesseur 16, puis converties en une seule séquence d'accélération selon l'axe de déplacement de la charge (généralement l'axe vertical) en employant la matrice de conversion de référentiel déterminée lors de la calibration.

Dans une variante, les données d'accélération selon les trois axes sont converties au fur et à mesure par le processeur ou même directement par des moyens logiques sur l'accéléromètre en une séquence d'accélération verticale stockée dans la mémoire. Une conversion des données avant stockage nécessite une puissance de calcul plus importante, mais permet de réduire la taille de la mémoire de stockage, ou d'augmenter la durée maximale d'acquisition possible pour un espace mémoire donné.

Au terme de l'acquisition, ou éventuellement déjà au cours de l'acquisition, le processeur 16 exécute une routine 209 de calcul d'au moins une grandeur représentative de la capacité musculaire de l'athlète. La grandeur calculée et le mode de calcul peuvent dépendre du type de test sélectionné au cours de l'étape 201. A titre d'exemple, les grandeurs suivantes peuvent être déterminées :

■ La puissance instantanée maximale ($\max(P_{inst})$) au cours de l'exercice ou d'une phase particulière de l'exercice (par exemple lors de la phase initiale d'accélération verticale lors d'un saut).

■ La puissance maximale pendant un intervalle de temps ($\Delta T$) de durée prédéterminée, par exemple la puissance maximale pendant n instants d'échantillonnages.

■ La puissance maximale au cours d'une phase du test prédéterminée entre deux instants clés $T_i$, par exemple la puissance développée lors de la poussée, lors d'une flexion, ou pour maintenir la charge levée.

■ Le taux maximal d'accroissement de la force TAF (indice d'explosivité), au cours de l'ensemble du test ou d'une phase déterminée du test.

■ La force maximale déployée selon la direction verticale, au cours de l'ensemble du test ou d'une phase déterminée du test.

■ L'énergie dépensée selon la direction verticale durant l'ensemble du test, ou durant une phase du test, par exemple en joules ou en calories.

■ La vitesse maximale atteinte durant l'ensemble du test, ou durant n'importe quelle phase du test.

■ La hauteur maximale atteinte lors d'un saut (tests II et III)

■ La durée de certaines phases du test, par exemple la durée de contact au sol lors d'un « drop jump », ou la durée de vol lors d'un saut.

■ Le rendement énergétique, par exemple sous forme de rapport entre le travail fourni par l'athlète et l'énergie cinétique de la charge déplacée.

■ La différence entre des valeurs mesurées ou calculées au terme de plusieurs tests, par exemple afin de déterminer la fatigue au cours d'un exercice répété, une disbalance entre muscles, une dissymétrie gauche-droite, une amélioration des performances de l'athlète, etc. La grandeur calculée au terme d'une série d'exercice peut par exemple être exprimée et affichée en pour-cents.

■ Etc

Il est aussi possible d'afficher plusieurs grandeurs au terme du test. Afin de réduire le volume et le poids du dispositif, on veillera cependant à employer un affichage numérique ou alphanumérique de petite dimension et à consommation réduite, par exemple un affichage alphanumérique à quatre lignes. L'affichage de multiples grandeurs peut alors être obtenu en naviguant entre plusieurs écrans de résultats.

Le calcul de certaines de ces grandeurs peut impliquer la détermination de la position temporelle de certains instants clés, et de la durée de la période entre deux instants clés. Des exemples d'instants clés T1 à T5 sont indiqués sur les figures 1 à 4 dans le cas d'un lever de charge. Dans le cas d'un drop jump, les instants clés seront par exemple le départ, le premier contact avec le sol, l'instant où l'athlète qui rebondit quitte le sol, l'instant où la hauteur maximale est atteinte, et l'instant où l'athlète rejoint pour la deuxième fois le sol après le rebond. La détermination automatique des instants clés à partir des données d'accélération dépend donc du type d'exercice sélectionné et de la connaissance a priori de la forme de la séquence de données d'accélération, ou d'au moins une portion de cette séquence. Fréquemment, les instants clés d'un mouvement sont des instants lors desquels l'accélération, la vitesse ou la position passent par un point particulier, par exemple un minimum, un maximum, un passage par zéro, un passage par une valeur particulière (par exemple 1G d'accélération) ou un point d'inflexion.

Il est aussi possible de déterminer les phases à partir desquelles les grandeurs affichées sont déterminées à partir du signe de certaines valeurs dérivées de l'accélération. Par exemple, on peut choisir de déterminer l'accélération maximale de tous les instants au cours desquels la vitesse est positive, c'est-à-dire orientée vers le haut. La détermination des instants-clés et des phases du mouvement peut aussi dépendre des données d'accélération selon des directions non verticales.

Les instants clés, et/ou la durée entre deux instants clés (« durée clé ») peuvent aussi être affichés au terme du test, comme grandeur supplémentaire représentative de la capacité musculaire de l'athlète.

La mesure peut être refusée par exemple si la forme de la séquence d'accélération obtenue ne correspond pas au modèle attendu selon le type de test sélectionné, et qu'elle ne permet pas d'obtenir les grandeurs recherchées. Dans ce cas, un signal d'erreur visuel et/ou sonore est de préférence émis pour inviter l'athlète à recommencer le test.

Au terme d'un test concluant, une ou plusieurs grandeurs ci-dessus sont immédiatement affichées au cours de l'étape 210 sur l'affichage 11. Plusieurs données peuvent être affichées sur plusieurs lignes ou sur plusieurs écrans entre lesquels l'utilisateur peut naviguer. Des grandeurs calculées peuvent aussi être mémorisées dans le dispositif pour comparaison avec des grandeurs calculées au cours d'autres tests.

L'utilisateur est ensuite invité à valider le test au cours de l'étape 211, par exemple en pressant une touche « ENTER » ou « OK ». S'il refuse de valider le test, il est invité au cours de l'étape 212 à recommencer en retournant à l'étape 203, ou à interrompre le test en passant directement à l'étape 213. Le programme passe directement à l'étape 213 lorsque l'utilisateur valide les résultats.

Au cours de l'étape 213, les résultats de tests validés sont stockés dans une mémoire semi-permanente 160, en les associant de préférence avec l'identification d'utilisateur, la date et l'heure du test, et une identification de type de test. Dans une première variante, adaptée à des dispositifs disposant d'une mémoire importante, toute la séquence de données d'accélération selon un axe, ou même selon les trois axes, est stockée en mémoire.

Cette variante permet de calculer ultérieurement d'autres grandeurs à partir de cette séquence, ou de la transférer via l'interface 162 et 19 vers une unité de traitement externe (PC, PDA, téléphone mobile, etc) pour exploitation ultérieure ou pour calculer et afficher d'autres grandeurs et d'autres graphiques. Le transfert peut être effectué automatiquement dès qu'une connexion est établie avec un dispositif externe, ou en sélectionnant une commande dans un menu ou par une autre action délibérée de l'utilisateur du dispositif 1.

Une application logicielle peut être chargée et exécutée dans le dispositif externe afin de calculer d'autres grandeurs, de les représenter différemment, d'en tirer des informations plus complètes que les informations de base immédiatement affichées sur le dispositif. Le logiciel permet par exemple d'afficher des grandeurs sous forme graphique, ou de les mettre en relation avec des mesures précédentes du même athlète, ou d'autres athlètes, ou avec les objectifs d'un plan d'entraînement qui peut être téléchargé depuis Internet. Cette application peut aussi effectuer des suggestions d'entraînement personnalisées en fonction des résultats de mesure de chaque athlète et en tenant compte des progrès de l'athlète en plusieurs tests datés. Le plan d'entraînement proposé peut tenir compte des résultats obtenus au cours de différents tests et à différentes dates.

Le logiciel permet aussi de préférence de classer et d'ordonner différents résultats de test à l'aide des métadonnées transmises par le dispositif, y compris la date et l'heure du test, l'identification d'athlète, l'identification de type de test, une identification éventuelle du dispositif employé, et/ou à l'aide de données supplémentaires introduites par l'opérateur pour classer et regrouper les résultats.

Les anciens tests qui ne sont plus pertinents ou qui ont été transférés sur l'unité de traitement externe peuvent de préférence être effacés de la mémoire du dispositif 1, soit automatiquement lors du transfert ou du stockage de nouveaux résultats, soit par une commande explicite introduite par l'utilisateur.

Dans le procédé ci-dessus, l'acquisition d'une nouvelle série de données d'accélération est reprise à chaque test, par exemple à chaque saut ou à chaque lever. Il est cependant fréquent d'effectuer un test plusieurs fois de suite et de prendre en compte uniquement le meilleur résultat. Par exemple, de nombreux entraîneurs sportifs recommandent de mesurer le « best squat jump », c'est-à-dire le meilleur saut dans une série de typiquement 3 sauts au cours d'une brève période. Si la mémoire du dispositif le permet, il est possible de programmer le dispositif de manière à saisir au cours d'une seule acquisition les données de plusieurs tests consécutifs ; le dispositif peut ensuite être programmé pour distinguer les trois sauts au cours de la séquence de mesure, et déterminer par exemple automatiquement le meilleur. Ce type de test permet en outre de déterminer à l'aide d'une seule séquence de données d'accélérations la fatigue ou au contraire le gain de détente entre tests consécutifs rapprochés, et d'afficher ainsi d'autres informations intéressantes relatives à la musculation de l'athlète.

La présente invention concerne aussi un système comprenant un dispositif de mesure 1 tel que décrit ci-dessus, associé à un support de données informatiques comprenant un programme informatique pour l'unité externe tel que ordinateur, PDA ou téléphone. Le support de donnée informatique peut être constitué par un CD-ROM, une mémoire semi-permanente (Flash, EEPROM, etc), un disque dur etc. Le programme informatique exécuté permet, comme indiqué plus haut, de communiquer avec le dispositif de mesure 1 et d'effectuer des calculs supplémentaires et d'afficher des informations additionnelles à partir des données reçues.

**Revendications**

1. Procédé d'évaluation des valeurs physiologiques musculaires d'athlètes (3) à l'aide de tests courts tels que levers et/ou sauts, comprenant les étapes suivantes :

fixation à une charge en mouvement (3 ; 2) au cours du test d'un dispositif de mesure (1) amovible et autonome électriquement, ledit dispositif de mesure comprenant un accéléromètre tri-axe (14),
détermination d'une séquence de valeurs d'accélération (a(t)) successive de ladite charge (3 ; 2) au cours dudit test,

immédiatement au terme dudit test, indication sur un affichage (11) du dispositif d'au moins une grandeur représentative de ladite capacité musculaire et déterminée à partir de ladite séquence de valeurs d'accélération.

2. Le procédé de la revendication 1, comprenant une étape de conversion de la séquence d'accélérations selon trois axes fournie par ledit accéléromètre tri-axe (14) en une séquence d'accélération selon la direction de déplacement présumée de ladite charge.

3. Le procédé de l'une des revendications 1 ou 2, comprenant une étape (201) de sélection initiale du type de test dans une liste comprenant au moins :

lever de charge ;
saut de l'athlète ;

la détermination de ladite grandeur affichée dépendant du choix du type de test.

4. Le procédé de la revendication 3, dans lequel ladite liste comporte au moins les tests suivants :

lever de charge ;
squat jump et/ou countermovement jump ;
drop jump.

5. Le procédé de l'une des revendications 1 à 4, comprenant une étape (201) d'introduction d'au moins un paramètre du test parmi les paramètres suivants :

poids de ladite charge,
hauteur de saut,
poids du sportif.

6. Le procédé de la revendication 5, dans lequel le paramètre à introduire dépend du type de test sélectionné.

7. Le procédé de l'une des revendications 3 à 6, dans lequel le choix de la grandeur affichée dépend du type de test sélectionné.

8. Le procédé de l'une des revendications 3 à 7, dans lequel le mode de calcul de la grandeur affichée dépend du type de test sélectionné.

9. Le procédé de l'une des revendications 1 à 8, dans lequel ladite grandeur affichée est déterminée en tenant compte d'une connaissance a-priori de la forme d'au moins une portion de ladite séquence d'accélération (a(t)).

10. Le procédé de l'une des revendications 1 à 9, dans lequel ladite au moins une grandeur affichée comprend au moins une grandeur proportionnelle à la puissance maximale déployée par ledit athlète (3) au cours du test.

**11.** Le procédé de la revendication 10, ladite puissance maximale étant une puissance instantanée (da/dt) maximale.

**12.** Le procédé de la revendication 10, ladite puissance maximale étant une puissance maximale pendant un intervalle de temps ($\Delta T$) de durée prédéterminée correspondant à plusieurs instants d'échantillonnages.

**13.** Le procédé de la revendication 10, ladite puissance maximale étant une puissance maximale pendant un intervalle de temps ($\Delta T$) entre deux instants clés ($T_i$) du test.

**14.** Le procédé de l'une des revendications 1 à 13, comportant une étape (209) de détermination automatique d'au moins un instant clé ($T_i$) ou une durée clé ($\Delta T$) du test à partir de ladite séquence de valeurs d'accélération (a(t)),
ladite grandeur affichée étant déterminée en tenant compte d'au moins un dit instant clé ou durée clé.

**15.** Le procédé de la revendication 14, dans lequel la détermination dudit instant clé ou de ladite durée clé dépend du type de test sélectionné.

**16.** Le procédé de la revendication 14, dans lequel ledit instant clé ($T_i$) ou ladite durée clé ($\Delta T$) sont affichés sur ledit affichage.

**17.** Le procédé de l'une des revendications 10 à 16, comprenant une étape de calcul (209) et d'affichage (210) au terme dudit test du taux maximal d'accroissement de la force.

**18.** Le procédé de l'une des revendications 1 à 17, dans lequel ladite au moins une grandeur affichée comprend au moins une grandeur proportionnelle à la vitesse maximale de ladite charge au cours du test.

**19.** Le procédé de l'une des revendications 1 à 18, dans lequel une séquence de valeurs d'accélération (a(t)) et/ou des données calculées à partir de ces valeurs d'accélération sont transmise à un dispositif de traitement externe pour calcul et affichage d'autres grandeurs ou graphiques représentatifs de ladite capacité musculaire.

**20.** Le procédé de l'une des revendications 1 à 19, comprenant une étape de détermination (204) de la direction verticale à l'aide des indications fournies par ledit accéléromètre tri-axial (14).

**21.** Le procédé de l'une des revendications 1 à 20, dans lequel ledit test est un saut, dans lequel un paramètre proportionnel à la masse (m) de l'athlète (3) est introduit dans ledit dispositif (1), dans lequel ledit dispositif (1) est fixé à la taille ou au tronc de l'athlète (3), dans lequel la direction verticale est déterminée avant le saut à partir des indications dudit accéléromètre (14) à l'arrêt avant le saut, et dans lequel ladite grandeur affichée vise à estimer l'explosivité de l'athlète.

**22.** Le procédé de l'une des revendications 1 à 21, dans lequel ledit test est un lever de charge (2), dans lequel un paramètre proportionnel à la charge levée est introduit dans ledit dispositif, dans lequel ledit dispositif est monté de manière amovible de manière à se déplacer avec ladite charge, dans lequel la direction verticale est déterminée avant le lever à partir des indications dudit accéléromètre à l'arrêt, et dans lequel ladite grandeur affichée vise à estimer la puissance de l'athlète.

**23.** Le procédé de l'une des revendications 1 à 22, dans lequel la durée dudit test est inférieure à 10 secondes, et dans lequel une dite valeur d'accélération est mesurée au moins tous les 1/100e de seconde.

**24.** Dispositif de mesure (1) pour la mise en oeuvre d'un procédé d'évaluation des valeurs physiologiques musculaires d'athlètes, comprenant:

des moyens de fixation amovible (12) permettant de fixer ledit dispositif à la charge déplacée (3 ; 2),
des moyens d'alimentation électrique autonomes (15),
un affichage (11)
un accéléromètre (14), pour fournir une séquence d'accélérations (a(t)) selon l'axe de déplacement de la charge, ladite séquence comprenant au moins 100 mesures par seconde pendant une durée comprise entre 1 et 10 secondes,
des moyens de traitement informatique (16), pour déterminer au terme du test, à partir de ladite séquence d'accélérations, au moins une grandeur représentative de la capacité musculaire de l'athlète et pour afficher cette grandeur sur ledit affichage (11),

charactérisé en que l'accéléromètre est tri-axial.

**25.** Le dispositif de la revendication 24, comprenant en outre des moyens de liaison amovibles (162, 19) vers un ordinateur, un PDA et/ou un ordinateur externe.

**26.** Le dispositif de l'une des revendications 24 ou 25, comprenant en outre des moyens de sélection du type d'exercice effectué parmi une liste de plusieurs exercices possibles.

27. Le dispositif de l'une des revendications 24 à 26, dans lequel lesdits moyens de fixation amovible (12) sont destinés à fixer ledit dispositif à la taille et/ou au tronc de l'athlète.

28. Le dispositif de la revendication 27, ledit accéléromètre tri-axe (14) ayant au moins un axe de mesure privilégié dont les résultats de mesure sont entachés d'une erreur moins importante que ceux du moins bon des deux autres axes,
lesdits moyens de fixation amovibles (12) étant agencés pour fixer ledit dispositif (1) en alignant substantiellement ledit axe de mesure privilégié avec la direction verticale.

29. Système comprenant un dispositif selon l'une des revendications 25 à 28 et un support de données informatiques comprenant un programme informatique destiné à être exécuté par une unité de traitement externe pour afficher des grandeurs supplémentaires à partir des données mesurées par ledit dispositif.

**Claims**

1. A method of evaluating muscular physiological parameters of athletes (3) using a short test, such as lifts and/or jumps, comprising the following steps:

   - a removable and electrically autonomous measurement device (1) is fastened to a weight (3; 2) that moves during said test, said measurement device comprising a three-axis accelerometer (14);
   - a sequence of successive accelerations (a(t)) of said weight (3; 2) is determined during said test;
   - immediately at the end of said test, at least one quantity representative of said muscular capacity, which is determined from said sequence of accelerations, is indicated on a display (11) of the device.

2. The method of claim 1, which includes a step of converting the sequence of accelerations along three axes, delivered by said three-axis accelerometer (14), into a sequence of accelerations along the presumed direction of movement of said weight.

3. The method of any of the claims 1 or 2, which includes a step (201) of initially selecting the type of test from a list comprising at least:

   - a weight lift;

   - a jump by the athlete,

the determination of said displayed quantity depending on the type of test chosen.

4. The method of claim 3, in which said list comprises at least the following tests:

   - a weight lift;
   - a squat jump and/or countermovement jump;
   - a drop jump.

5. The method of any of the claims 1 to 4, which includes a step (201) of inputting at least one parameter of the test from the following parameters:

   - the mass of said weight;
   - the jump height;
   - the weight of the sportsman.

6. The method of claim 5, in which the parameter to be input depends on the type of test selected.

7. The method of any of the claims 3 to 6, in which the choice of quantity displayed depends on the type of test selected.

8. The method of any of the claim 3 to 7, in which the method of calculation of the displayed quantity depends on the type of test selected.

9. The method of any of the claims 1 to 8, in which said displayed quantity is determined by taking into account *a priori* knowledge of the form of at least one portion of said sequence of accelerations (a(t)).

10. The method of any of the claims 1 to 9, in which said at least one displayed quantity comprises at least one quantity proportional to the maximum power deployed by said athlete (3) during the test.

11. The method of claim 10, said maximum power being an instantaneous maximum power (da/dt).

12. The method of claim 10, said maximum power being a maximum power during a time interval ($\Delta$T) of predetermined duration corresponding to several sampling instants.

13. The method of claim 10, said maximum power being a maximum power during a time interval ($\Delta$T) between two key instants ($T_i$) of the test.

14. The method of any of the claims 1 to 13, which includes a step (209) of automatically determining at least one key instant ($T_i$) or a key duration ($\Delta$T) of the test from said sequence of accelerations (a(t)), said displayed quantity being determined by taking into account at least one said key instant or key duration.

15. The method of claim 14, in which the determination of said key instant or said key duration depends on the type of test selected.

16. The method of claim 14, in which said key instant ($T_i$) or said key duration ($\Delta T$) is displayed on said display.

17. The method of any of the claims 10 to 16, which includes after said test a step (209) of calculation (209) and display (210) of the maximum force enhancement.

18. The method of any of the claims 1 to 17, in which said at least one displayed quantity comprises at least one quantity proportional to the maximum velocity of said weight during the test.

19. The method of any of the claims 1 to 18, in which a sequence of accelerations (a(t)) and/or of data calculated from these accelerations is transmitted to an external processing device for calculating and displaying other quantities or graphs representative of said muscular capacity.

20. The method of any of the claims 1 to 19, which includes a step (204) of determining the vertical direction using indications provided by said three-axis accelerometer (14).

21. The method of any of the claims 1 to 20, in which said test is a jump, in which a parameter proportional to the mass (m) of the athlete (3) is input into said device (1), in which said device (1) is fastened to the waist or trunk of the athlete (3), in which the vertical direction is determined before the jump on the basis of indications from said accelerometer (14) at rest before the jump, and in which said displayed quantity is aimed at estimating the explosive power of the athlete.

22. The method of any of the claims 1 to 21, in which said test is a weight lift (2), in which a parameter proportional to the weight lifted is input into said device, in which said device is removably fitted so as to move with said weight, in which the vertical direction is determined before the lift on the basis of indications from said accelerometer at rest, and in which said displayed quantity is aimed at estimating the power of the athlete.

23. The method of any of claims 1 to 22, in which the duration of said test is less than 10 seconds and in which a said acceleration is measured at least every 100th of a second.

24. A test device (1) for operation of a evaluation method of physiological muscular values of athletes, comprising:

- removable fastening means (12) for fastening said device to a moved weight (3; 2);
- autonomous electrical power supply means (15);
- a display (11);
- a accelerometer (14) for delivering a sequence of accelerations (a(t)) along the axis of movement of said weight, said sequence comprising at least 100 measurements per second over a duration between 1 and 10 seconds; and
- data processing means (16) for determining, at the end of the test, on the basis of said sequence of accelerations, at least one quantity representative of the muscular capacity of the athlete and for displaying this quantity on said display (11),

**characterized in that** the accelerometer (14) is three-axis.

25. The device of claim 24, which further includes removable means (162, 19) for linking to a computer, a PDA and/or an external computer.

26. The device of any of the claims 24 or 25, which further includes means for selecting the type of exercise performed from a list of several possible exercises.

27. The device of any of the claims 24 to 26, in which said removable fastening means (12) are intended for fastening said device to the athlete's waist and/or trunk.

28. The device of claim 27, said three-axis accelerometer (14) having at least one preferential measurement axis whose measurement results are affected by a smaller error than those of the worst of the two other axes, said removable fastening means (12) being designed to fasten said device (1) by aligning said preferential measurement axis substantially with the vertical direction.

29. System comprising a device according to one of the claims 25 to 28 and a data processing medium, comprising a computer program intended to be executed by an external processing unit for displaying additional quantities on the basis of the data measured by said device.

**Patentansprüche**

1. Ein Verfahren, um muskulär pysiologische Parameter eines Athleten (3) mit der Hilfe von kurzen Tests wie Gewichtheben und/oder Sprüngen zu bestimmen, umfassend die folgenden Schritte:

Befestigung eines Gewichts (3; 2), welches sich während des Tests bewegt, an eine entfernbare und elektrisch autonome Vorrichtung (1), wobei besagte Messvorrichtung ein dreiachsiges Beschleunigungsmesser (14) umfasst,
Bestimmen einer Sequenz von Beschleunigungswerten (a(t)) des besagten Gewichts (3; 2) während des Tests; und

sofort nach dem Ende des Tests Darstellung auf einer Anzeige (11) der Vorrichtung mindestens eine Quantitätsdarstellung der muskulären Kapazität, welche aufgrund von besagter Sequenz von Beschleunigungen bestimmt wurde.

**2.** Das Verfahren gemäss Anspruch 1, umfassend einen Schritt Umwandeln der Sequenz von Beschleunigungen entlang drei Achsen, die durch besagtes dreiachsiges Beschleunigungsmessgerät (14) abgegeben werden, in eine Sequenz von Beschleunigungen entlang der angenommenen Bewegungsrichtung von besagtem Gewicht.

**3.** Das Verfahren gemäss Anspruch 1 oder 2, umfassend einen Schritt (201) von anfänglicher Auswahl der Art des Tests von einer Liste, umfassend mindestens:

Gewichtheben;
einen Sprung des Athleten;

wobei die Bestimmung die Bestimmung der besagten Quantität von der Art des ausgewählten Tests abhängt;

**4.** Das Verfahren gemäss Anspruch 3, in welchem besagte Liste mindestens folgende Tests umfasst:

Gewichtheben;
einen Squat jump und/oder einen Counter-Movement-Jump;
Dropjump.

**5.** Das Verfahren gemäss einem der Ansprüche 1 bis 4, umfassend einen Schritt (201) von Eingabe mindestens eines Testparameters von den folgenden Parametern:

Masse des besagten Gewichts,
Sprunghöhe,
Gewicht des Sportlers.

**6.** Das Verfahren gemäss Anspruch 5, in welchem der einzugebende Parameter von der Art des ausgewählten Tests.

**7.** Das Verfahren gemäss einem der Ansprüche 3 bis 6, in welchem die Auswahl der angezeigten Quantität von der Art des ausgewählten Tests abhängt.

**8.** Das Verfahren gemäss einem der Ansprüche 3 bis 7, in welchem die Berechnung der angezeigten Quantität von der Art des ausgewählten Tests abhängt.

**9.** Das Verfahren gemäss einem der Ansprüche 1 bis 8, in welchem besagte angezeigte Quantität durch Berücksichtigung eines a priori Wissens der Form von mindestens einem Teil von besagter Sequenz von Beschleunigungen (a(t)) bestimmt wird.

**10.** Das Verfahren gemäss einem der Ansprüche 1 bis 9, in welchem besagte mindestens eine angezeigte Quantität mindestens eine Quantität proportional zu der maximalen Leistung, die von dem Athleten (3) eingesetzt wird, umfasst.

**11.** Das Verfahren gemäss Anspruch 10, wobei besagte maximale Leistung eine spontane maximal Leistung (da/dt) ist.

**12.** Das Verfahren gemäss Anspruch 10, wobei besagte maximale Leistung während eines Zeitintervalls ($\Delta T$) von einer vorherbestimmten Dauer, die mehreren Beispielmomenten entspricht, ist.

**13.** Das Verfahren gemäss Anspruch 10, wobei besagte maximale Leistung eine maximale Leistung während eines Zeitintervalls ($\Delta T$) zwischen zwei Schlüsselmomenten ($T_i$) des Tests ist.

**14.** Das Verfahren gemäss einem der Ansprüche 1 bis 13, umfassend einen Schritt (209) der automatischen Bestimmung von mindestens einem Schlüsselmoment ($T_i$) oder einer Schlüsselzeitdauer ($\Delta T$) des Tests von besagter Sequenz von Beschleunigungen (a(t)),
wobei besagte angezeigte Quantität unter Berücksichtigung von mindestens besagtem Schlüsselmoment oder der Schlüsseldauer bestimmt wird.

**15.** Das Verfahren gemäss Anspruch 14, in welchem die Bestimmung des besagten Schlüsselmoments oder besagter Schlüsseldauer von der Art des ausgewählten Tests abhängt.

**16.** Das Verfahren gemäss Anspruch 14, in welchem besagter Schlüsselmoment ($T_i$) oder besagte Schlüsseldauer ($\Delta T$) auf der Anzeige dargestellt wird.

**17.** Das Verfahren gemäss einem der Ansprüche 10 bis 16, umfassend einen Berechnungsschritt (209) und einen Anzeigeschritt (210), um nach dem Test die maximale Krafterweiterung zu berechnen und anzuzeigen.

18. Das Verfahren gemäss einem der Ansprüche 1 bis 17, in welchem besagte mindestens eine angezeigte Quantität mindestens eine Quantität proportional zu der maximalen Geschwindigkeit von besagtem Gewicht umfasst.

19. Das Verfahren gemäss einem der Ansprüche 1 bis 18, in welchem die Sequenz von Beschleunigungen (a(t)) und/oder von Daten, die aus diesen Beschleunigungen berechnet wurden, zu einer externen Verarbeitungseinheit übermittelt werden, um andere Quantitäten oder Graphiken, die besagte muskuläre Kapazität repräsentieren, zu berechnen und anzuzeigen.

20. Das Verfahren gemäss einem der Ansprüche 1 bis 19, welches einen Schritt von Bestimmen der vertikalen Richtung unter Verwendung von Anzeichen des besagten dreiachsigen Beschleunigungsmessgeräts (14) umfasst.

21. Das Verfahren gemäss einem der Ansprüche 1 bis 20, in welchem besagter Test ein Sprung ist, in welchem ein Parameter proportional zu der Masse (m) des Athleten (3) in besagte Vorrichtung (1) eingegeben wird, in welchem besagte Vorrichtung (1) an der Taille oder dem Rumpf des Athleten (3) angebracht wird, in welchem die vertikale Richtung des Sprungs auf der Basis von Anzeichen von besagtem Beschleunigungsmesser (14) in einer Ruhestellung vor dem Sprung bestimmt wird, und in welchem besagte angezeigte Quantität beabsichtigt, die explosive Leistung des Athleten zu schätzen.

22. Das Verfahren gemäss einem der Ansprüche 1 bis 21, in welchem besagter Test ein Gewichtsheben ist, in welchem ein Parameter proportional zu dem gehobenen Gewicht in besagte Vorrichtung (1) eingegeben wird, in welchem besagte Vorrichtung entfernbar angepasst wird, um sich mit dem Gewicht zu bewegen, in welchem die vertikale Bewegung von dem Heben auf der Basis von Anzeichen von dem Beschleunigungsmesser in einer Ruheposition bestimmt wird, und in welchem besagte anzeigte Quantität beabsichtigt, die Leistung des Athleten zu schätzen.

23. Das Verfahren gemäss einem der Ansprüche 1 bis 22, in welchem die Dauer des besagten Tests kleiner als 10 Sekunden ist, und in welchem eine besagte Beschleunigung mindestens alle 100stel einer Sekunde gemessen wird.

24. Eine Testvorrichtung (1) zur Durchführung eines Verfahrens zur Bestimmung von mulkulären pysiologischen Werten eines Athleten, umfassend:

Entfernbare Befestigungsmittel (12), um besagte Vorrichtung an ein bewegtes Gewicht (3; 2) zu befestigen;
eine autonome elektrische Stromversorgung (15);
eine Anzeige (11);
einen Beschleunigungsmesser, **dadurch gekennzeichnet, dass** der Beschleunigungsmesser (14) dreiachsig ist, um eine Sequenz von Beschleunigungen (a(t)) entlang der Bewegungsachse des Gewichts abzugeben, besagte Sequenz umfassend mindestens 100 Messungen pro Sekunde über eine Dauer von 1 und 10 Sekunden,
Datenverarbeitungsmittel (16), um am Ende des Tests auf der Basis von besagter Sequenz von Beschleunigungen mindestens eine Quantitätsdarstellung der muskulären Kapazität des Athleten zu bestimmen und dann diese Quantität auf besagtem Display (11) anzuzeigen.

25. Die Testvorrichtung gemäss Anspruch 24, weiter umfassend entfernbare Mittel (162, 19) um einen Computer, einen PDA und/oder einen externen Computer anzuschliessen.

26. Die Testvorrichtung gemäss Anspruch 24 oder 25, weiter umfassend Mittel, um die Art des ausgewählten Tests von einer Liste aus verschiedenen Übungen auszuwählen.

27. Die Testvorrichtung gemäss einem der Ansprüche 24 bis 26, in welcher besagte entfernbare Befestigungsmittel dazu da sind, um besagte Vorrichtung an der Taille und/oder dem Rumpf des Athleten zu befestigen.

28. Die Testvorrichtung gemäss Anspruch 27, in welchem besagtes dreiachsiges Beschleunigungsmesser (14) mindestens eine bevorzugte Achse hat, von der die Messergebnisse von einem kleineren Fehler beeinflusst werden als die weniger guten anderen beiden Achsen, besagte entfernbare Befestigungsmittel (12) sind durch Fluchten von besagter bevorzugter Messachse im Wesentlichen mit der vertikalen Richtung ausgestaltet.

29. System umfassend eine Vorrichtung, wie sie in einem der Ansprüche 25 bis 28 beansprucht wird, und ein Datenverarbeitungsmedium, umfassend ein Computerprogramm, welches gedacht ist, um durch eine externe Verarbeitungseinheit ausgeführt zu werden, um zusätzliche Quantitäten auf der Basis von durch besagte Vorrichtung gemessenen Daten anzuzeigen.

Fig. 1

Fig. 2

Fig. 3

Fig. 4

EP 1 834 583 B1

P max

F max
V max
P max

F max

V max

m [Kg]

Fig. 5

a

(~f)

$\dfrac{da}{dt}$

$\Delta a$

a max

$$TAF = \dfrac{dF}{dt} \sim \dfrac{da}{dt}$$

T0   T1

$\Delta t$

t

Fig. 6

Fig. 7

Fig. 8

**Fig. 9**

**Fig. 10**

Fig. 11

Fig. 12

Fig. 13

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5788655 A, Omron
- WO 2005074795 A, Nokia
- WO 03032826 A, Philips
- US 5474083 A
- US 6148280 A
- DE 446302
- US 6397151 B